# EUROPÄISCHE PATENTSCHRIFT

(11) **EP 3 150 345 B1**
(45) Veröffentlichungstag und Bekanntmachung des Hinweises auf die Patenterteilung: **14.03.2018**
(21) Anmeldenummer: 15187355.1
(22) Anmeldetag: 29.09.2015
(51) Int. Cl.: B27N 1/00, B27N 3/12, B27K 5/00

(54) **HOLZFASERDÄMMSTOFFE MIT REDUZIERTER EMISSION AN FLÜCHTIGEN ORGANISCHEN VERBINDUNGEN (VOCS) UND VERFAHREN ZU DEREN HERSTELLUNG**
WOOD FIBER INSULATING MATERIALS WITH REDUCED EMISSION OF VOLATILE ORGANIC COMPOUNDS (VOCS) AND METHOD FOR THEIR PREPARATION
MATERIAUX ISOLANTS EN FIBRES DE BOIS PRESENTANT UNE FAIBLE EMISSION DE COMPOSES ORGANIQUES VOLATILES (COV) ET SON PROCEDE DE FABRICATION

(43) Veröffentlichungstag der Anmeldung: 05.04.2017
(73) Patentinhaber: SWISS KRONO Tec AG, 6004 Luzern (CH)
(72) Erfinder: DR. KALWA, Norbert, 32805 Horn-Bad Meinberg (DE); SIEMS, Jens, 17139 Malchin (DE)
(74) Vertreter: Maikowski & Ninnemann Patentanwälte Partnerschaft mbB

(56) Entgegenhaltungen:
- EP-A1- 1 674 224
- WO-A2-2012/168563
- FR-A1- 2 989 016

## Beschreibung

Die vorliegende Erfindung betrifft ein Verfahren zur Herstellung von Holzfaserdämmstoffmatten gemäß dem Oberbegriff nach Anspruch 1, mit dem Verfahren hergestellte Holzfaserdämmstoffmatten gemäß Anspruch 10 und die Verwendung von torrefizierten Holzfasern gemäß Anspruch 13.

### Beschreibung

Holzfaserdämmstoffe erfreuen sich aufgrund ihrer Nachhaltigkeit steigender Beliebtheit zum Beispiel als Gefachdämmung, Innenraumdämmung beim Hausbau oder als Trittschall mindernde Lage zwischen Geschossdecke und Bodenbelag.

Allerdings besteht bei der Verwendung von Holzwerkstoff- bzw. Holzdämmstoffmatten insbesondere in Innenräumen derzeit eine noch gesundheitliche Belastungsquelle aufgrund der Emission von während des Herstellungsprozesses der Holzfasern gebildeten leicht flüchtigen organischen Verbindungen (VVOCs) oder flüchtigen organischen Verbindungen (VOCs). Hierzu gehören organische Stoffe, die leicht verdampfen bzw. bereits bei niedrigen Temperaturen, wie z.B. bei Raumtemperatur gasförmig vorliegen.

Die wichtigsten Prozesse bei denen diese Substanzen freigesetzt werden bzw. entstehen sind die Zerfaserung des Holzes, Trocknung der Holzfasern und Pressung der Holzfaserdämmstoffe. In all diesen Prozessen wird das Holz bzw. werden die Holzfasern hohen Temperaturen in Kombination mit sehr niedrigen pH-Werten unterworfen, wobei aus den verschiedenen Holzbestandteilen (Lignin, Zellulose und Hemizellulose) die Substanzen direkt oder durch chemische Umwandlung freigesetzt werden. Aufgrund der geringen Rohdichte von Holzfaserdämmstoffen können die Holzinhaltstoffe leicht entweichen und können zu Geruchsbelästigungen in der Nutzung der Holzfaserdämmstoffmatten führen.

Das Problem der Emission von flüchtigen organischen Komponenten aus Holzfaserplatten, insbesondere von Aldehyden, ist umso gravierender, je niedriger die Dichte der hergestellten Holzwerkstoffplatten ist. Während bei einer Faserplatte mit erhöhter Dichte (HDF) oder einer mitteldichten Faserplatte (MDF) keine erhöhten Werte der leichtflüchtigen organischen Bestandteile ermittelbar sind, ist bei Holzfaserplatten unterhalb einer Rohdichte von ca. 250 kg/m³, wie z.B. dem Holzfaserdämmstoffmatten, eine erhebliche VOC-Emission zu verzeichnen, da hier aufgrund der geringen Dichte Diffusionsvorgänge beschleunigt ablaufen können. Vor allem dies trägt zur schnellen Abgabe von organischen Verbindungen aus den Dämmmaterialien bei.

Um das Problem der VOC-Emission zu lösen wurden in der Vergangenheit verschiedene Ansätze beschrieben. Zum einen besteht die Möglichkeit Holzfasern mit anderen natürlichen Fasern wie z.B. Wolle, Hanfflachs, zu mischen, die sich im Hinblick auf ihr Emissionsverhalten günstiger verhalten, um somit einen ökologischen Dämmstoff mit verbesserter Emissionscharakteristik zu erhalten. Ein Nachteil hierbei ist allerdings die mit diesen Fasern verbundenen hohen Kosten und eingeschränkte Verfügbarkeit, da teilweise für die entsprechenden Faserarten auch höherwertige Anwendungen existieren, die einen anderen Einsatz nahelegen.

Auch kann durch Zugabe von alkalischen Stoffen der pH-Wert in der Holzmatrix erhöht werden, um so die in der Holzmatrix ablaufenden säurekatalysierten Reaktionen zu verhindern bzw. zu reduzieren (Roffael, E., et al, Holzzentralblatt 1990, 116: 1684-1685). Weitere Möglichkeiten in der Reduzierung der Emission von leichtflüchtigen organischen Verbindung bestehen in der Zugabe von Zeolith (WO 2010/ 136106), Bisulfiten oder Pyrosulfiten (US2009/0130 474 A1) als Aldehydfänger oder auch in der Zugabe von Polyaminen zur Reduzierung von während des wässrigen Holzaufschlusses freigesetzten Aldehyden und organischen Säuren (EP 2 567 798).

Aus den oben genannten Gründen ist es daher erstrebenswert, weitergehende Lösungen zu entwickeln, durch welche die Freisetzung von leichtflüchtigen organischen Verbindungen aus Holzwerkstoffplatten, insbesondere aus Holzfaserdämmstoffmatten reduziert wird.

Die EP 1674224 B1 beschreibt die Herstellung von Holzfaserdämmstoffplatten in einem großen Dicke-und Rohdichtebereich. Hier werden Holzfasern und Bindefasern vermischt und unter räumlicher Ausrichtung zu einer Matte gelegt, die anschließend von heißer Luft durchströmt wird, was zur Vernetzung mit hervorragenden Querzugseigenschaften der hergestellten Holzfaserdämmstoffplatten führt.

Der vorliegenden Erfindung liegt nunmehr die technische Aufgabe zugrunde, ein Verfahren zur Herstellung von Holzfaserdämmstoffmatten bereitzustellen, welches die Herstellung von Holzfaserdämmstoffmatten mit einer reduzierten Emission von flüchtigen organischen Verbindungen ermöglicht. Dabei soll der zur Herstellung von Holzfaserdämmstoffmatten verwendete Prozess so wenig wie möglich verändert werden und eine zu starke Kostensteigerung der Produkte vermieden werden.

Entsprechend lag der vorliegenden Erfindung die technische Aufgabe zugrunde, einen geeigneten Rohstoff zu finden, der zur Herstellung von Holzfaserdämmstoffmatten geeignet ist und in Kombination mit Holzfasern einsetzbar ist sowie ein günstiges Emissionsverhalten aufweist.

Diese Aufgabe wird erfindungsgemäß durch ein Verfahren zur Herstellung von Holzfaserdämmstoffmatten mit den Merkmalen des Anspruchs 1 und eine daraus hergestellte Holzfaserdämmstoffmatte gemäß Anspruch 10 gelöst.

Entsprechend wird ein Verfahren zur Herstellung von Holzfaserdämmstoffmatten, insbesondere von Holzfaserdämmstoffmatten mit reduzierter Emission an flüchtigen organischen Verbindungen (VOCs), bereitgestellt, welches die folgenden Schritte umfasst:
a) Herstellen von Holzhackschnitzeln (oder Holzstrands) aus geeigneten Hölzern,
b) Torrefizieren von zumindest einem Teil der Holzhackschnitzel;
c) Faseraufschluss der unbehandelten Holzhackschnitzel und zumindest eines Teils der torrefizierten Holzhackschnitzel zu Holzfasern;
d) Vermischen der torrefizierten Holzfasern oder eines Gemisches aus unbehandelten und torrefizierten Holzfasern mit einem Bindemittel;
e) Aufbringen, z.B. Aufstreuen oder Aufblasen, der Mischung aus Holzfasern und Bindemittel auf ein Transportband unter Ausbildung eines Faserkuchens; und
f) Erwärmen und Verdichten des Faserkuchens zu einer Holzfasermatte.

Das vorliegende Verfahren ermöglicht die Herstellung von Holzfaserdämmstoff bzw.

Holzfaserdämmstoffmatten unter Verwendung von torrefiziertem Holz, insbesondere torrefizierten Holzhackschnitzeln, die zusätzlich oder alternativ zu unbehandelten Holzhackschnitzeln in einen bekannten Herstellungsprozess eingeführt werden. Eine mit dem erfindungsgemäßen Verfahren hergestellte Holzfaserdämmstoffmatte umfassend torrefiziertes Holz weist eine verminderte Emission von flüchtigen organischen Verbindungen, insbesondere von Essigsäure und Furfural auf.

Torrefizierung ist ein thermochemisches Behandlungsverfahren, bei welchem das zu torrefizierende Material in einer sauerstoffreduzierten oder sauerstofffreien Gasatmosphäre bei Atmosphärendruck erhitzt wird. Aufgrund des Mangels an Sauerstoff verbrennt das Material nicht, stattdessen kommt es zu einem Masseverlust aufgrund der Eliminierung von Gasen und einer sogenannten Torrefizierung. Torrifiziertes Material ist hydrophob und daher weniger anfällig gegenüber der Umgebungsfeuchte, so dass die Verrottungsgefahr von torrefizierten Material äußerst gering ist.

Der Torrifizierungsschritt der Holzhackschnitzel kann in dem vorliegenden Verfahren in verschiedener Weise vorgesehen sein.

So ist es gemäß einer ersten Ausführungsform möglich, den Schritt des Torrefizierens der Holzhackschnitzel in den Herstellungsprozess der Holzfaserdämmstoffmatten zu integrieren, d.h. der Torrefizierungsschritt ist in den Gesamtprozess bzw. Prozesslinie eingegliedert und erfolgt online.

In einer zweiten Ausführungsvariante kann der Schritt des Torrefizierens der Holzhackschnitzel separat von dem Herstellungsprozess der Holzfaserdämmstoffmatten durchgeführt werden. Demnach erfolgt der Torrefizierungsschritt in dieser Ausführungsvariante des vorliegenden Verfahrens außerhalb des Gesamtprozesses bzw. der Prozesslinie. Die Holzhackschnitzel werden hierbei aus dem Herstellungsprozess ausgeschleust und in die Torrefizierungsvorrichtung (z.B. Torrefizierungsreaktor) eingeführt. Anschließend können die torrefizierten Holzhackschnitzel ggf. nach einer Zwischenlagerung wieder in den herkömmlichen Herstellungsprozess eingeschleust werden. Dies ermöglicht eine hohe Flexibilität im Herstellungsverfahren.

Die vorliegend verwendeten Holzhackschnitzel können eine Länge zwischen 10 bis 100 mm, bevorzugt 20 bis 90 mm, insbesondere bevorzugt 30 bis 80 mm; eine Breite zwischen 5 bis 70 mm, bevorzugt 10 bis 50 mm, insbesondere bevorzugt 15 bis 20 mm; und eine Dicke zwischen 1 und 30 mm, bevorzugt zwischen 2 und 25 mm, insbesondere bevorzugt zwischen 3 und 20 mm aufweisen.

Der Torrefizierungsschritt umfasst bevorzugt das Erhitzen der Holzhackschnitzel in sauerstoffarmer oder sauerstofffreier Atmosphäre unter Atmosphärendruck, wobei Sattdampf oder Inertgas, bevorzugt Stickstoff als Reaktionsgas oder Gasstrom verwendet wird. Die Holzhackschnitzel werden bei einer Temperatur zwischen 200 und 300°C, bevorzugt zwischen 220 und 280°C, insbesondere bevorzugt zwischen 240 und 260°C torrefiziert.

Der Prozess des Torrefizierens wird bevorzugt bei einem Masseverlust der Holzhackschnitzel von 10 bis 30%, bevorzugt 15 bis 20% beendet. Die Dauer des Prozesses variiert in Abhängigkeit der Menge und Art des eingesetzten Ausgangsmaterials und kann zwischen 1 und 5 h, bevorzugt zwischen 2 und 3 h betragen.

Der vorliegend zum Einsatz kommende Torrefizierungsreaktor kann als Batch-Anlage oder als kontinuierlich betriebene Anlage vorliegen.

Die während des Torrefizierungsprozesses im Wesentlichen aus Hemicellulosen und anderen niedermolekularen Verbindungen freigesetzten Pyrolysegase werden zur Erzeugung von Prozessenergie benutzt. Dabei ist die Menge an gebildeten Gasgemisch als gasförmiger Brennstoff ausreichend, um, den Prozess energetisch zu betreiben.

Als vorteilhaft hat sich zudem ein Vortrocknen der Holzhackschnitzel auf eine Feuchte von 5 bis 15%, bevorzugt 5 bis 10% (in Bezug auf die Ausgangsfeuchte) erwiesen.

Die torrefizierten Holzhackschnitzel werden bevorzugt auf Raumtemperatur abgekühlt und ggf. zwischengelagert oder dem Herstellungsprozess unmittelbar, ggf. nach Befeuchtung, wieder zugeführt.

In einer weiteren Ausführungsform des vorliegenden Verfahrens werden die unbehandelten und zumindest ein Teil der torrefizierten Holzhackschnitzel einer Wasserbehandlung unterzogen, wobei die Feuchte der torrefizierten Hackschnitzel auf 5 bis 20%, bevorzugt 10 bis 15% eingestellt wird. Die Holzhackschnitzel werden in diesem Schritt z.B. gewaschen und gekocht. Die Wasserbehandlung ist wünschenswert, damit sich die Holzhackschnitzel zerfasern lassen. Zudem würde ohne Wasser bei der Zerfaserung sehr viel unerwünschter Staub entstehen.

Es schließt sich ein Zerfaserungsprozess der Holzhackschnitzel in einem Refiner an, wobei den Holzfasern während des Zerfaserungsprozesses mindestens ein Benetzungsmittel zugegeben werden kann. Das Benetzungsmittel verbessert die Benetzung insbesondere des torrefizierten Holzes bzw. der Holzhackschnitzel mit Wasser.

Die mit dem Zerfaserungsprozess hergestellten Holzfasern weisen eine Länge zwischen 1,5 mm und 20 mm und eine Dicke zwischen 0,05 mm und 1 mm auf.

In einem weiteren Schritt des vorliegenden Verfahrens werden die Holzfasern nach dem Zerfaserungsprozess mit mindestens einem zur Vernetzung der Holzfasern geeigneten Bindemittel kontaktiert.

Das In-Kontaktbringen der Holzfasern mit dem mindestens einen Bindemittel in Schritt d) erfolgt bevorzugt in einem Blow-Line-Verfahren, bei dem das Bindemittel in den Holzfaserstrom eingespritzt wird. Hierbei ist es möglich, dass die beschriebenen Bindemittel zur Holzfaservernetzung in der Blow-Line einem Holzfaser-Dampfgemisch zugeführt werden. Im Falle der Verwendung von Bindemittel-Fasern werden diese hingegen den trockenen Holzfasern zugemischt; wie weiter unten erläutert.

Es ist ebenfalls vorstellbar, dass mehr als ein Bindemittel zur Vernetzung der Holzfasern verwendet werden. So können Gemische aus natürlichen und synthetisch biologisch abbaubaren Bindemitteln verwendet werden.

Es ist auch denkbar, das Bindemittel zur Vernetzung der Holzfasern während des Dämpfens der Hackschnitzel einzuführen oder im Refiner zuzugeben. Dabei können 5 bis 20 Gew%, bevorzugt 10 bis 15 Gew % an Bindemittel auf die Holzfasern aufgetragen werden.

Es ist aber auch denkbar, das Bindemittel mittels Trockenbeleimung mit den Holzfasern in Kontakt zu bringen. Das Bindemittel wird hier durch extrem feines Verdüsen auf die getrockneten Holzfasern aufgebracht. Eine derartige Trockenbeleimung reduziert den Leimverbrauch gegenüber einer Blow-Line-Beleimung drastisch.

Als geeignetes Bindemittel für das Holzfasergemisch können formaldehydhaltige Harze wie Melaminharze, Isocyanate oder aber Kunststofffasern eingesetzt werden.

Bevorzugt ist das weitere Bindemittel für das Holzfasergemisch mindestens ein Isocyanat, ausgewählt aus einer Gruppe enthaltend aliphatische und aromatische Isocyanate. Typischerweise können als aliphatische Isocyanate z.B. Hexamethylendiisocyanat (HDI), Isophorondiisocyanat (IPDI) und/oder 1,4-Cyclohexyldiisocyanat (CHDI) verwendet werden. Als bevorzugte aromatische Polyisocyanate können Polymeres Diphenylmethandiisocyanat (PMDI) Toluylendiisocyanat (TDI) und/oder Diphenylmethandiisocyanat (MDI) zum Einsatz kommen, wobei PMDI besonders bevorzugt ist. Das Isocyanat unterliegt bei seiner Verwendung als Bindemittel zwei chemischen Reaktionen. Zum einen bildet es in Gegenwart von Wasser Polyharnstoff aus. Parallel erfolgt die Anbindung an die Holzfasern durch Ausbildung einer Urethan-Bindung durch die Reaktion von Isocyanate mit den freien HydroxyGruppen der Zellulose.

Als Bindemittel für das Holzfasergemisch können aber auch Kunststofffasern zum Einsatz kommen. Die Kunststofffasern können als Monokomponenten-Fasern oder als Bikomponenten-Fasern vorliegen. Die Vermischung der Holzfasern mit derartigen Bindefasern erfolgt bevorzugterweise in einer Blasleitung vor der Vliesbildung, wie weiter unten noch im Detail beschrieben wird.

Werden Monokomponenten-Fasern verwendet, bestehen diese bevorzugt aus Polyethylen oder anderen Kunststoffen mit einem niedrigen Schmelzpunkt.

Bikomponentenfasern (auch als BiCo-Stützfasern bezeichnet) bestehen typischerweise aus einem Tragfilament oder aus einer Kernfaser aus einem Kunststoff mit höherer Temperaturbeständigkeit, insbesondere Polypropylen, die aus einem Kunststoff mit einem niedrigeren Schmelzpunkt, insbesondere aus Polyethylen, umhüllt bzw. ummantelt ist. Die Hülle bzw. der Mantel ermöglicht nach Aufschmelzen eine Vernetzung der Holzfasern.

Das zur Herstellung der Holzfaserdämmstoffmatte verwendete Fasergemisch kann 60 bis 90 Gew%, bevorzugt 70 bis 80 Gew% an Holzfasern und 5 bis 20 Gew%, bevorzugt 10 bis 15 Gew% an Bindemitteln umfassen.

In einer Ausführungsform umfasst das vorliegend verwendete Fasergemisch zwischen 10 und 50 Gew%, bevorzugt zwischen 20 und 30 Gew% unbehandelte Fasern und zwischen 50 und 90 Gew%, bevorzugt zwischen 70 und 80 Gew% an torrefizierten Fasern.

Es ist ebenfalls möglich, zusammen oder separat mit dem Bindemittel den Holzfasern mindestens ein Flammschutzmittel zuzuführen.

Das Flammschutzmittel kann typischerweise in einer Menge zwischen 1 und 10 Gew%, bevorzugt zwischen 3 und 7 Gew%, insbesondere bevorzugt 5 Gew% dem Holzfaser-Bindemittel-Gemisch zugegeben werden.

Typische Flammschutzmittel sind ausgewählt aus der Gruppe umfassend Phosphate, Borate, insbesondere Ammoniumpolyphosphat, Tris(tri-bromneopentyl)phosphat, Zinkborat oder Borsäurekomplexe von mehrwertigen Alkoholen.

Nach der optionalen Zugabe eines Bindemittels in den Holzfaserstrom und dem Beleimen der Holzfasern erfolgt ein Trocknen der Holzfasern zu einem Feuchtegrad von 1 bis 10 %, bevorzugt 3 bis 5 %, wobei dieser Trocknungsprozess in zwei Stufen ausgeführt werden kann.

Die getrockneten Holzfasern werden anschließend entsprechend ihrer Größe sortiert bzw. gesichtet und bevorzugterweise zwischengelagert, zum Beispiel in Silos oder Bunkern.

Wie oben bereits erwähnt, ist es möglich, anstatt eines flüssigen Bindemittels Bindefasern als Bindemittel einzusetzen. In diesem Falle werden die Bindefasern aus Ballenöffnern bereitgestellt und die notwendige Menge an Bindefasern bestimmt.

Die Bindefasern werden mit den Holzfasern auf ein erstes Transportband unter Ausbildung eines Vorvlieses aufgebracht, insbesondere aufgeblasen. Gemäß dieser Variante des Herstellungsverfahrens können die Holzfasern und das Bindemittel z.B. in Form von Bindefasern über einer Blasleitung geführt und auf ein erstes Transportband aufgeblasen werden. In der Blasleitung erfolgt dabei eine intensive Vermischung von Holzfasern und Bindemittel (Bindefasern) und gegebenenfalls weiteren Komponenten durch die eingeblasene Luft als Transportmittel. Die Menge an zugeführtem Fasergemisch richtet sich nach der gewünschten Schichtdicke und der gewünschten Rohdichte der herzustellenden Holzfasermatte.

Nach Ablage der Mischung aus Holzfasern und Bindemittel und gegebenenfalls weiteren Komponenten auf dem ersten Transportband können vorliegend unter Verwendung eines Pulverstreuers (Hersteller: z.B. Fa. TPS) weitere Komponenten wie z.B. ein thermisch aktivierbares Kunststoffgranulat auf das gebildete Vorvlies aufgestreut werden. Das thermisch aktivierbare Kunststoffgranulat kann in Mengen von 5-45 Gew%, bevorzugt in Mengen von 10-40 Gew%, insbesondere bevorzugt in Mengen von 20-30 Gew% bezogen auf das jeweils eingesetzte Fasergemisch zugegeben werden. Gut geeignet als Kunststoffgranulate sind solche, die beim Recyceln von Kunststoffartikeln aus dem dualen System anfallen.

In einem weitergehenden Schritt des vorliegenden Verfahrens wird das auf dem ersten Transportband abgelegte Vorvlies zerfasert, vermischt und die Fasermischung auf ein zweites Transportband unter Ausbildung eines Faserkuchens aufgebracht. Die Dicke des erhaltenen Faserkuchens bzw. Matte wird dabei durch die Umlaufgeschwindigkeit des zweiten Transportbandes eingestellt.

Wie oben bereits ausgeführt, wird der Faserkuchen anschließend erwärmt und verpresst bzw. verdichtet, wobei die hierfür verwendeten Temperaturen zwischen 100°C und 250°C, bevorzugt 130°C und 220°C, insbesondere bei 200°C liegen. Mit dem Verdichten und/oder Erwärmen ist eine Aktivierung des Bindemittels bzw. der Bindefasern in der Holzfasermatte verbunden.

In der Endbearbeitung wird die Fasermatte schließlich auf die gewünschten Maße reduziert und gekühlt; die Kühlung erfolgt bevorzugt während der Kalibrierung und in der Kühlzone des Durchlaufofens.

In einer besonders bevorzugten Ausführungsform umfasst das vorliegenden Verfahrens zur Herstellung einer Holzfaserdämpfstoffmatte mit reduzierter VOC-Emission die folgenden Schritte:
a1) Herstellen von Holzhackschnitzeln (oder Holzstrands) aus geeigneten Hölzern,
b1) ggf. Vortrocknen der Holzhackschnitzel,
c1) Torrefizieren von zumindest einem Teil der Holzhackschnitzel,
d1) Wasserbehandlung der torrefizierten Holzhackschnitzel,
e1) Faseraufschluss der unbehandelten Holzhackschnitzel und zumindest eines Teiles der torrefizierten Holzhackschnitzel zu Holzfasern;
f1) Vermischen der torrefizierten Holzfasern oder einer Mischung aus unbehandelten Holzfasern und torrefizierten Holzfasern mit Bindefasern, insbesondere Bikomponentenfasern;
g1) Aufbringen, z.B. Aufstreuen oder Aufblasen, der Mischung aus Holzfasern und Bindefasern auf ein erstes Transportband unter Ausbildung eines Vorvlieses,
h1) Zerfasern des Vorvlieses, Vermischung und Aufbringen der Fasermischung auf ein zweites Transportband unter Ausbildung eines Faserkuchens,
i1) Erwärmen und Verdichten des Faserkuchens zu einer Holzfaserdämmstoffmatte.

Die Verwendung von torrefizierten Holzhackschnitzeln zur Herstellung von Holzfaserdämmstoffmatten weist eine Reihe von Vorteilen auf. So ist es besonders vorteilhaft, dass die aus den torrefizierten Holzhackschnitzeln hergestellten torrefizierten Holzfasern besonders leicht zu trocknen sind, was in der geringen Hydrophilie der torrefizierten Holzfasern begründet ist. Dies ist auch für die Nutzung der aus den torrefizierten Holzfasern hergestellten Dämmstoffe von Vorteil, da die torrefizierten Holzfasern bei definierten Temperaturen und Luftfeuchten eine niedrigere Ausgleichsfeuchte besitzen als nicht torrifizierte Holzfasern. Entsprechend kann ein Befall der Holzfaserdämmstoffe mit Mikroorganismen wie zum Beispiel mit Schimmelpilzen während der Nutzung weitestgehend ausgeschlossen werden.

Ein weiterer positiver Aspekt der Verwendung von torrefizierten Holzhackschnitzeln ist, dass eine Vergleichmäßigung des Ausgangsrohstoffes Holz erreicht wird. Dies ist von besonderer wirtschaftlicher Bedeutung, da beim Einsatz von Holzhackschnitzeln zur Herstellung von Holzfaserdämmstoffen oder anderen Holzwerkstoffen die jahreszeitlichen Schwankungen des Rohstoffes Holz berücksichtigt werden müssen. Ein weiterer Vorteil ist, dass torrefizierte Holzhackschnitzel keinem biologischen Abbau oder anderen Änderungen durch Lagerung unterworfen sind, wodurch eine Lagerung der torrefizierten Holzhackschnitzeln über einen längeren Zeitraum möglich ist. Des Weiteren werden keine Inhaltsstoffe durch Wasserkontakt ausgewaschen, da diese im Torrefizierungsprozess zerstört worden sind.

Entsprechend ermöglicht das vorliegende Verfahren die Herstellung einer Holzfaserdämmstoffmatte mit reduzierter Emission an flüchtigen organischen Verbindungen (VOCs), welche torrefizierte Holzfasern umfasst. Die vorliegende Holzfaserdämmstoffmatte kann dabei vollständig aus torrefizierten Holzfasern bestehen oder aus einem Gemisch von unbehandelten (d.h. nicht torrefizierten) Holzfasern und torrefizierten Holzfasern bestehen.

Die vorliegende Holzfaserdämmstoffmatte weist insbesondere eine reduzierte Emission von während des Holzaufschlusses freigesetzten Aldehyden, insbesondere Furfural, von organischen Säuren, insbesondere Essigsäure, und/oder von Terpenen auf. Diesbezüglich wird auch auf die Ausführungen weiter unten verwiesen.

Die vorliegende Holzfaserdämmstoffmatte kann eine Rohdichte zwischen 20 und 170 kg/m³, bevorzugt zwischen 50 und 150 kg/m³, insbesondere bevorzugt zwischen 100 und 140 kg/m³ aufweisen.

Die Dicke der vorliegenden Holzfaserdämmstoffmatte kann zwischen 3 und 20 mm, bevorzugt zwischen 5 und 15 mm betragen, wobei insbesondere eine Dicke von 5 mm bevorzugt ist.

Die vorliegende Holzfaserdämmstoffmatte besteht aus einem Fasergemisch umfassend 60 bis 90 Gew%, bevorzugt 70 bis 80 Gew% an Holzfasern und 5 bis 20 Gew%, bevorzugt 10 bis 15 Gew% an Bindemitteln, insbesondere Bindefasern. Diesbezüglich wird auf die obigen Ausführungen zur Art der verwendeten Bindemittel verwiesen.

Wie oben ausgeführt, kann die vorliegende Holzfaserdämmstoffmatte aus einem Gemisch aus unbehandelten Holzfasern und torrefizierten Holzfasern bestehen. Das in der Holzfaserdämmstoffmatte verwendete Fasergemisch kann zwischen 10 und 50 Gew%, bevorzugt zwischen 20 und 30 Gew% unbehandelte Fasern und zwischen 50 und 90 Gew%, bevorzugt zwischen 70 und 80 Gew% an torrefizierten Fasern umfassen.

Die Aufgabe der vorliegenden Erfindung wird ebenfalls mit der Verwendung von torrefizierten Holzfasern gemäß Anspruch 13 gelöst.

Demnach werden torrefizierte Holzfasern zur Reduzierung der Emission von flüchtigen organischen Verbindungen (VOCs) aus Holzfaserdämmstoffmatten verwendet. Die torrefizierten Holzfasern werden bevorzugt aus torrefizierten Holzhackschnitzeln gewonnen.

In einer bevorzugten Variante werden die torrefizierten Holzfasern zur Reduzierung von während des Holzaufschlusses freigesetzten Aldehyden, organischen Säuren und/oder Terpenen verwendet.

Entsprechend werden die torrefizierten Holzfasern vorliegend bevorzugt zur Reduzierung der Emission von organischen Säuren, insbesondere zur Reduzierung der Emission von Essigsäure aus Holzwerkstoffmatten verwendet. Organische Säuren fallen insbesondere als Spaltprodukte der Holzbestandteile Zellulose, Hemizellulosen und Lignin an, wobei bevorzugt Alkansäuren, wie Essigsäure und Propionsäure oder aromatische Säuren gebildet werden.

Es ist ebenfalls wünschenswert, die torrefizierten Holzfasern zur Reduzierung der Emission von Aldehyden aus Holzwerkstoffmatten einzusetzen. Hierbei ist es insbesondere bevorzugt, wenn die torrefizierten Holzfasern zur Reduzierung von während des wässrigen Holzaufschlusses freigesetzten Aldehyden eingesetzt wird. Wie oben bereits erläutert, erfolgt eine Freisetzung von Aldehyden während der hydrolytischen Aufarbeitung von Holz bzw. Lignozellulose. Dabei können spezifische Aldehyde aus den Grundbausteinen der Zellulose oder Hemizellulose gebildet werden. So wird z.B. der Aldehyd Furfural aus Mono-und Disacchariden der Zellulose bzw. Hemizellulose gebildet, während aromatische Aldehyde während des partiell stattfindenden hydrolytischen Ausschlusses von Lignin freigesetzt werden können. Entsprechend werden die torrefizierten Holzfasern zur Reduzierung der Emission von C1-C10 Aldehyden, insbesondere bevorzugt vom Formaldehyd, Acetaldehyd, Pentanal, Hexanal oder auch Furfural in Holzfaserdämmstoffmatten eingesetzt.

In einer weiteren Ausführungsform der vorliegenden Erfindung werden die torrefizierten Holzfasern zur Reduzierung der Emission von Terpenen verwendet. So können die torrefizierten Holzfasern zur Reduzierung von aus üblicherweise in der Holzfaserdämmstoffmatte verwendeten Holzspänen bzw. Holzfasern freigesetzten Terpenen, insbesondere C10-Monoterpene und C15-Sesquiterpene, insbesondere bevorzugt acyclische oder cyclische Monoterpene eingesetzt werden.

Typische acyclische Terpene sind Terpenkohlenwasserstoffe wie Myrcen, Terpenalkohole wie Gerianol, Linaool, Ipsinol und Terpenaldehyde wie Citral. Typische Vertreter der monocyclischen Terpene sind p-Menthan, Terpeninol, Limonen oder Carvon, und typische Vertreter der bicyclischen Terpene sind Caran, Pinan, Bornan, wobei insbesondere 3-Caren und α-Pinen von Bedeutung sind. Terpene sind Bestandteile der Baumharze und von daher besonders in sehr harzhaltigen Baumarten wie Kiefer oder Fichte vorhanden.

Die Erfindung wird nachfolgend unter Bezugnahme auf die Figur der Zeichnung an einem Ausführungsbeispiel näher erläutert. Es zeigt:

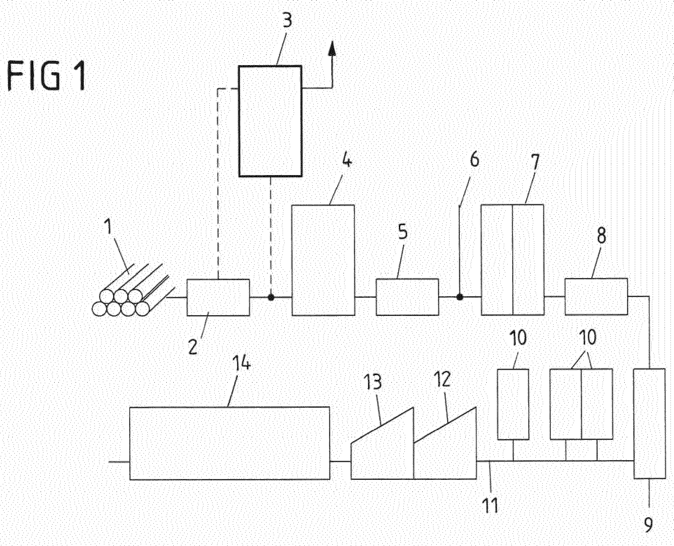

- Figur 1: eine schematische Darstellung einer Ausführungsform des erfindungsgemäßen Verfahrens.

Die in Figur 1 gezeigte Ausführungsform des erfindungsgemäßen Verfahrens beschreibt die einzelnen Verfahrensschritte beginnend mit dem bereitstellen des Holzausgangsproduktes bis zur fertigen Holzfaserdämmstoffmatte.

Entsprechend wird zunächst in Schritt 1 geeignetes Holzausgangsmaterial zur Herstellung der Holzhackschnitzel bereitgestellt. Als Holzausgangsmaterial sind sämtliche Nadelhölzer, Laubhölzer oder auch Mischungen davon geeignet.

Die Zerkleinerung des Holzausgangsmaterials gemäß Schritt 2 erfolgt in hierfür geeigneten Häckselmaschinen, wobei die Größe der Holzhackschnitzel bzw. Holzstrands entsprechend gesteuert werden kann. Nach Zerkleinerung und Bereitstellung der Holzhackschnitzel werden diese ggf. einem Vortrocknungsprozess unterzogen, wobei eine Feuchte von 5-10 % in Bezug auf die Ausgangsfeuchte der Holzhackschnitzel eingestellt wird.

Im Falle der in Figur 1 gezeigten Ausführungsform wird zumindest ein Teil der vorgetrockneten Holzhackschnitzel aus dem üblichen Herstellungsverfahren ausgeschleust und in einen Torrefizierungsreaktor eingeführt (Schritt 3). Die Torrefizierung der ausgeschleusten Holzhackschnitzel erfolgt in einem Temperaturbereich zwischen 220° und 260°C. Die dabei entstehenden Pyrolysegase werden zur Erzeugung der für die Prozessanlage notwendigen Energie genutzt.

Nach Abschluss der Torrefizierung, die im vorliegenden Fall ca. 2 Stunden dauert, werden die torrefizierten Holzhackschnitzel in das Verfahren wieder eingeschleust und werden gegebenenfalls zusammen mit den unbehandelten Holzhackschnitzeln in einem Wasch- und Kochschritt 4 wieder auf eine Feuchte von 10-20 % gebracht.

Danach werden die Holzfasern dem Zerfaserungsprozess in einem Refiner (Schritt 5) unterworfen, wobei im Verlaufe des Zerfaserungsprozesses den Holzfasern ein geeignetes Benetzungsmittel zugeführt wird.

Die Holzfasern können unmittelbar nach dem Faseraufschluss mit einem flüssigen Bindemittel und gegebenenfalls einem Flammschutzmittel vermischt werden (Schritt 6). Das In-Kontaktbringen der Holzfasern mit dem flüssigen Bindemittel kann in dieser Verfahrensstufe zum Beispiel in einem Blow-line- Verfahren erfolgen.

Dem optionalen Beleimungsschritt 6 schließt sich ein Trocknungsprozess der beleimten Holzfasern (Schritt 7) an, wobei dieser Trocknungsprozess in zwei Stufen I, II erfolgen kann. Der Trockner ist als 2 Stufen Trockner ausgeführt, wobei die hauptsächliche Trocknung in Stufe 1 mittels heißer Gase (Luft oder überhitzter Dampf) erfolgt und Nachtrocknung in Stufe 2, wobei hier ebenfalls der Einsatz von heißer Luft oder überhitzten Dampf möglich ist. Das Stoffgemisch wird in/nach jeder Stufe mittels Abscheidezyklon und Kapselwerke getrennt.

Die getrockneten Holzfasern werden entsprechend ihrer Größe sortiert bzw. gesichtet (Schritt 8) und in geeigneten Vorratsbehältern (zum Beispiel Silo oder Bunker) zwischengelagert (Schritt 9).

Anschließend werden die Holzfasern mit Bikomponentenfasern als Bindefasern vermischt. Hierfür werden die Bikomponentenfasern aus Ballenöffnern (Schritt 10) bereitgestellt und die notwendige Menge an Bikomponentenfasern bestimmt.

Zum Vermischen der Bikomponentenfasern mit den Holzfasern werden diese über eine Blasleitung (Schritt 11) geführt und auf ein erstes Transportband aufgeblasen. In der Blasleitung (11) erfolgt eine intensive Vermischung von Holzfasern und Bikomponentenfasern durch die eingeblasene Luft als Transportmittel.

Durch Ablage der Holzfaser-Bikomponentenfasern-Mischung auf dem ersten Transportband kommt es zur Ausbildung eines vor Vorvlieses (Schritt 12). Das Vorvlies wird am Ende des ersten Transportbandes erneut zerfasert, vermischt und die Fasermischung auf ein zweites Transportband unter Ausbildung eines Faserkuchens aufgebracht (Schritt 13). Die Kombination der Bildung eines Vorvlieses mit erneuter Zerfaserung ist auch als Air-lay-Verfahren bekannt.

Der Faserkuchen wird anschließend bei einer Temperatur von ca. 200 °C verdichtet (Schritt 14) und auf die gewünschte Dicke eingestellt. Hierbei durchströmt heiße Luft den Faserkuchen und es kommt zu einer Aktivierung der Bindefasern in der Holzfasermatte, so dass die erwärmten und angeschmolzenen Bindefasern sich mit den Holzfasern verbinden.

In der Endbearbeitung wird die erhaltene Holzfasermatte in geeigneter Weise konfektioniert.

### Ausführungsbeispiel:

Handelsübliche Holzhackschnitzel getrocknet oder nicht getrocknet werden ohne weitere Vorbereitung einer Torrefizierungsapparatur zu geführt. Diese kann kontinuierlich oder in einem Batch-Verfahren funktionieren.

Die Holzhackschnitzel werden dann in einem Torrefizierungsprozess auf eine Temperatur von 240°C erhitzt. Dies erfolgt entweder unter Sattdampf oder einer Inertgas-Atmosphäre (Stickstoff). Der Prozess braucht ca. 2 Stunden, wobei sich die Hackschnitzel von hellgelb nach mittel- bis dunkelbraun verfärben. Nachdem ein Masseverlust von ca. 15 - 20% erreicht worden ist, werden die Hackschnitzel in einem Wasserbad abgekühlt und dann in einem Refiner zu Holzfasern aufgeschlossen. Zur besseren und schnelleren Benetzung kann ein Netzmittel zugegeben werden Dabei werden die üblichen Prozessparameter verwendet wie bei nicht torrefizierten Hackschnitzeln.

Aus den Holzfasern und BiCo-Fasern werden in einer Anlage zur Herstellung von Dämmstoffen Holzfaserdämmstoffe produziert. Der Anteil der BiCo-Fasern lag dabei ca. 10 %.

Aus dieser Produktion wurde ein Muster zur Bestimmung von VOC's (volatile organic compounds = flüchtige organische Bestandteile) gezogen. Eine Nullprobe aus der Standardproduktion wurde als Vergleich mitgeprüft.

Die Ergebnisse nach 7 Tagen sind wie folgt, wobei nur die mengenmäßig wichtigsten Parameter angeben werden:

| **Parameter** | **Nullprobe (µg/m³)** | **Prüfmuster (µg/m³)** |
|---|---|---|
| Essigsäure | 1073 | 22 |
| Furfural | 57 | 3 |
| Benzaldehyd | 11 | 4 |

Wie aus den Prüfergebnissen zu entnehmen ist, liegen die Emissionen des Prüfmusters auf einem deutlich niedrigeren Niveau als die Nullprobe.

## Patentansprüche

1. Verfahren zur Herstellung von Holzfaserdämmstoffmatten, insbesondere von Holzfaserdämmstoffmatten mit reduzierter Emission an flüchtigen organischen Verbindungen (VOCs), umfassend die Schritte:
a) Herstellen von Holzhackschnitzeln aus geeigneten Hölzern,
b) Torrefizieren von zumindest einem Teil der Holzhackschnitzel;
c) Faseraufschluss der unbehandelten Holzhackschnitzel und zumindest eines Teiles der torrefizierten Holzhackschnitzel zu Holzfasern;
d) Vermischen der torrefizierten Holzfasern oder eines Gemisches aus unbehandelten und torrefizierten Holzfasern mit mindestens einem Bindemittel;
e) Aufbringen der Mischung aus Holzfasern und Bindemittel auf ein Transportband unter Ausbildung eines Faserkuchens; und
f) Erwärmen und Verdichten des Faserkuchens zu einer Holzfasermatte.

2. Verfahren nach Anspruch 1, **dadurch gekennzeichnet**, das der Schritt des Torrefizierens der Holzhackschnitzel in den Herstellungsprozess der Holzfaserdämmstoffmatten integriert ist.

3. Verfahren nach Anspruch 1, **dadurch gekennzeichnet, dass** der Schritt des Torrefizierens der Holzhackschnitzel separat von dem Herstellungsprozess der Holzfaserdämmstoffmatten durchgeführt wird.

4. Verfahren nach einem der vorhergehenden Ansprüche, **dadurch gekennzeichnet, dass** die Holzhackschnitzel durch Erhitzen in sauerstoffarmer oder sauerstofffreier Atmosphäre unter Atmosphärendruck bei einer Temperatur zwischen 200°C und 300°C, bevorzugt zwischen 220°C und 280°C, insbesondere bevorzugt zwischen 240°C und 260°C torrefiziert werden.

5. Verfahren nach einem der vorhergehenden Ansprüche, **dadurch gekennzeichnet, dass** die Feuchte der torrefizierten Hackschnitzel auf 5 bis 20%, bevorzugt 10 bis 15% eingestellt wird.

6. Verfahren nach einem der vorhergehenden Ansprüche, **dadurch gekennzeichnet, dass** die Holzfasern nach dem Zerfaserungsprozess mit mindestens einem zur Vernetzung der Holzfasern geeigneten Bindemittel kontaktiert werden.

7. Verfahren nach einem der vorhergehenden Ansprüche, **dadurch gekennzeichnet, dass** die Holzfasern mit Bindefasern auf ein erstes Transportband unter Ausbildung eines Vorvlieses aufgebracht, insbesondere aufgeblasen werden.

8. Verfahren nach Anspruch 7, **dadurch gekennzeichnet, dass** das Vorvlies zerfasert, vermischt und die Faservermischung auf ein zweites Transportband unter Ausbildung eines Faserkuchens aufgebracht wird.

9. Verfahren nach einem der vorhergehenden Ansprüche **dadurch gekennzeichnet, dass** der Faserkuchen bei Temperaturen zwischen 100°C und 250°C, bevorzugt 130°C und 220°C, insbesondere bei 200°C verdichtet wird.

10. Holzfaserdämmstoffmatte mit reduzierter Emission an flüchtigen organischen Verbindungen (VOCs) herstellbar in einem Verfahren nach einem der vorhergehenden Ansprüche umfassend torrefizierte Holzfasern.

11. Holzfaserdämmstoffmatte nach Anspruch 10, **gekennzeichnet durch** eine reduzierte Emission von während des Holzaufschlusses freigesetzten Aldehyden, organischen Säuren und/oder Terpenen.

12. Holzfaserdämmstoffmatte nach Anspruch 10 oder 11 **dadurch gekennzeichnet, dass** diese vollständig aus torrefizierten Holzfasern bestehen oder aus einem Gemisch von unbehandelten Holzfasern und torrefizierten Holzfasern besteht.

13. Verwendung von torrefizierten Holzfasern zur Reduzierung der Emission von flüchtigen organischen Verbindungen (VOCs) aus Holzfaserdämmstoffmatten.

14. Verwendung von torrefizierten Holzfasern nach Anspruch 13, **dadurch gekennzeichnet, dass** die Holzfasern aus torrefizierten Holzhackschnitzeln erhalten werden.

15. Verwendung von torrefizierten Holzfasern nach Anspruch 13 oder 14 zur Reduzierung von während des Holzaufschlusses freigesetzten Aldehyden, organischen Säuren und/oder Terpenen.

## Claims

1. Process for the production of wood-fibre insulation mats, in particular of wood-fibre insulation mats with reduced emission of volatile organic compounds (VOCs), comprising the steps of:
a) production of wood chips from suitable types of wood,
b) torrefaction of at least a portion of the wood chips;
c) fibre-extractive destructurization of the untreated wood chips and of at least a portion of the torrefied wood chips to give wood fibres;
d) mixing of the torrefied wood fibres or of a mixture of untreated and torrefied wood fibres with at least one binder;
e) application of the mixture of wood fibres and binder to a conveyor belt with formation of a fibre cake; and
f) heating and compaction of the fibre cake to give a wood-fibre mat.

2. Process according to Claim 1, **characterized in that** the step of the torrefaction of the wood chips has been integrated into the process for producing the wood-fibre insulation mats.

3. Process according to Claim 1, **characterized in that** the step of the torrefaction of the wood chips is carried out separately from the process for producing the wood-fibre insulation mats.

4. Process according to any of the preceding claims, **characterized in that** the wood chips are torrefied by heating in a low-oxygen-content or oxygen-free atmosphere under atmospheric pressure at a temperature of from 200°C to 300°C, preferably from 220°C to 280°C, with particular preference from 240°C to 260°C.

5. Process according to any of the preceding claims, **characterized in that** the moisture content of the torrefied wood chips is adjusted to from 5 to 20%, preferably from 10 to 15%.

6. Process according to any of the preceding claims, **characterized in that**, after the defibration process, the wood fibres are brought into contact with at least one binder suitable for the crosslinking of the wood fibres.

7. Process according to any of the preceding claims, **characterized in that** the wood fibres are applied to, in particular blown onto, a first conveyor belt with binding fibres, with formation of a preliminary web.

8. Process according to Claim 7, **characterized in that** the preliminary web is defibrated and mixed and the fibre mixture is applied to a second conveyor belt with formation of a fibre cake.

9. Process according to any of the preceding claims, **characterized in that** the fibre cake is compacted at temperatures of from 100°C to 250°C, preferably from 130°C to 220°C, in particular at 200°C.

10. Wood-fibre insulation mat which has reduced emission of volatile organic compounds (VOCs) and which can be produced in a process according to any of the preceding claims comprising torrefied wood fibres.

11. Wood-fibre insulation mat according to Claim 10, **characterized by** reduced emission of terpenes and/or organic acids and/or aldehydes liberated during wood digestion.

12. Wood-fibre insulation mat according to Claim 10 or 11, **characterized in that** this consists entirely of torrefied wood fibres or of a mixture of untreated wood fibres and torrefied wood fibres.

13. Use of torrefied wood fibres to reduce the emission of volatile organic compounds (VOCs) from wood-fibre insulation mats.

14. Use of torrefied wood fibres according to Claim 13, **characterized in that** the wood fibres are obtained from torrefied wood chips.

15. Use of torrefied wood fibres according to Claim 13 or 14 to reduce quantities of terpenes and/or organic acids and/or aldehydes liberated during wood digestion.

## Revendications

1. Procédé servant à fabriquer des panneaux en matériau isolant à base de fibres de bois, en particulier des panneaux en matériau isolant à base de fibres de bois présentant des émissions réduites de composés organiques volatiles (VOC), comprenant les étapes consistant à :
a) fabriquer des copeaux de bois à partir de bois adaptés ;
b) torréfier au moins une partie des copeaux de bois ;
c) désagréger les fibres des copeaux de bois non traités et d'au moins une partie des copeaux de bois torréfiés en des fibres de bois ;
d) mélanger les fibres de bois torréfiées ou un mélange composé des fibres de bois non traitées et torréfiées avec au moins un liant ;
e) appliquer le mélange composé de fibres de bois et de liant sur une bande transporteuse en formant un gâteau de fibres ; et
f) réchauffer et compacter le gâteau de fibres en un panneau à base de fibres de bois.

2. Procédé selon la revendication 1, **caractérisé en ce que** l'étape consistant à torréfier les copeaux de bois est intégrée dans le processus de fabrication des panneaux en matériau isolant à base de fibres de bois.

3. Procédé selon la revendication 1, **caractérisé en ce que** l'étape consistant à torréfier les copeaux de bois est effectuée de manière séparée du processus de fabrication des panneaux en matériau isolant à base de fibres de bois.

4. Procédé selon l'une quelconque des revendications précédentes, **caractérisé en ce que** les copeaux de bois sont torréfiés par un chauffage sous une atmosphère pauvre en oxygène ou sans oxygène, à une pression atmosphérique, à une température comprise entre 200 °C et 300 °C, de manière préférée entre 220 °C et 280 °C, en particulier de manière préférée entre 240 °C et 260 °C.

5. Procédé selon l'une quelconque des revendications précédentes, **caractérisé en ce que** l'humidité des copeaux de bois torréfiés est réglée à 5 à 20 %, de manière préférée à 10 à 15 %.

6. Procédé selon l'une quelconque des revendications précédentes, **caractérisé en ce que** les fibres de bois sont mises en contact après le processus de défibrage avec au moins un liant adapté pour la réticulation des fibres de bois.

7. Procédé selon l'une quelconque des revendications précédentes, **caractérisé en ce que** les fibres de bois comprenant des fibres de liaison sont appliquées, en particulier sont soufflées, sur une première bande transporteuse en formant une première couche de fibres.

8. Procédé selon la revendication 7, **caractérisé en ce que** la première couche de fibres est défibrée, mélangée, et le mélange de fibres est appliqué sur une deuxième bande transporteuse en formant un gâteau de fibres.

9. Procédé selon l'une quelconque des revendications précédentes, **caractérisé en ce que** le gâteau de fibres est compacté à des températures comprises entre 100 °C et 250 °C, de manière préférée entre 130 °C et 220 °C, en particulier à une température de 200 °C.

10. Panneau en matériau isolant à base de fibres de bois présentant des émissions réduites de composés organiques volatiles (VOC), pouvant être fabriqué lors d'un procédé selon l'une quelconque des revendications précédentes, comprenant des fibres de bois torréfiées.

11. Panneau en matériau isolant à base de fibres de bois selon la revendication 10, **caractérisé par** des émissions réduites d'aldéhydes libérés au cours de la désagrégation de bois, d'acides organiques et/ou de terpènes.

12. Panneau en matériau isolant à base de fibres de bois selon la revendication 10 ou 11, **caractérisé en ce qu'**il est constitué en totalité de fibres de bois torréfiées ou d'un mélange de fibres de bois non traitées et de fibres de bois torréfiées.

13. Utilisation de fibres de bois torréfiées servant à réduire les émissions de composés organiques volatiles (VOC) provenant des panneaux en matériau isolant à base de fibres de bois.

14. Utilisation de fibres de bois torréfiées selon la revendication 13, **caractérisée en ce que** les fibres de bois sont obtenues à partir de copeaux de bois torréfiés.

15. Utilisation de fibres de bois torréfiées selon la revendication 13 ou 14 servant à réduire des aldéhydes libérés au cours de la désagrégation du bois, des acides organiques et/ou des terpènes.
